# FASCICULE DE BREVET EUROPEEN

(11) **EP 1 846 063 B1**
(45) Date de publication et mention de la délivrance du brevet: **27.02.2019**
(21) Numéro de dépôt: 05823672.0
(22) Date de dépôt: 21.12.2005
(51) Int. Cl.: A61M 5/145

(54) **DISPOSITIF DE RETENUE POUR BLOQUER LA TÊTE DE PISTON D'UNE SERINGUE SUR LE POUSSOIR D'UN POUSSE SERINGUE**
HALTEVORRICHTUNG ZUM ARRETIEREN DES KOPFES EINES SPRITZENKOLBENS AUF EINEM SPRITZENPUMPENSCHIEBER
HOLDING DEVICE FOR LOCKING THE HEAD OF A SYRINGE PISTON ON A SYRINGE PUMP PUSHER

(30) Priorité: 17.01.2005 FR 0550132
(43) Date de publication de la demande: 24.10.2007
(73) Titulaire: Fresenius Vial SAS, 38590 Brézins (FR)
(72) Inventeur: ROCHETTE, François, 38140 Apprieu (FR)
(74) Mandataire: Vièl, Frédérique
(86) Numéro de dépôt international: PCT/EP2005/057032
(87) Numéro de publication internationale: WO 2006/074858

(56) Documents cités:
- EP-A- 1 195 172
- EP-A- 1 279 410
- WO-A1-01/08726
- US-A1- 2004 116 893

## Description

L'invention concerne un dispositif de retenue pour bloquer la tête de piston d'une seringue sur le poussoir d'un pousse seringue, le dispositif étant muni de bras solidaires chacun d'un pignon pouvant pivoter autour de son axe et pouvant se déplacer en translation en entraînant le bras correspondant dans un même mouvement entre une position de repos dans laquelle les bras sont « fermés » et « plaqués » contre le poussoir, et une position d'ouverture dans laquelle les bras sont « ouverts » et « éloignés » du poussoir, la translation étant réalisée avant la rotation lors du mouvement vers la position d'ouverture ou inversement vers la position de repos, le dispositif de retenue comprenant en outre un organe de commande et des moyens pour transformer une partie du mouvement de l'organe de commande en le transmettant à au moins un pignon sous la forme d'un mouvement de translation et des moyens pour transformer une partie du mouvement de l'organe de commande en le transmettant à au moins un pignon sous la forme d'un mouvement de rotation.

Il est courant dans le milieu médical d'utiliser des pousse seringues. Ces pousses seringues sont constitués essentiellement d'un dispositif pour fixer le cylindre de la seringue et d'un dispositif pour pousser le piston dans le cylindre à une vitesse définie afin de délivrer de façon contrôlée la solution au patient. Par ailleurs, il est préférable que la tête de piston soit retenue contre le poussoir du pousse seringue pour éviter un phénomène de siphonage. Il arrive parfois qu'une dépression se forme en aval de la seringue. Si la tête de piston n'est pas retenue, la seringue risque de se vider partiellement ou entièrement en délivrant au patient la solution médicamenteuse à un rythme beaucoup trop important, ce qui peut avoir des conséquences dramatiques.

Différentes solutions ont été proposées. Par exemple, on connaît du document EP 1 279 410 A1 un dispositif de retenue actionné à la main comprenant un élément mobile perpendiculairement à l'axe de la seringue. Cet élément mobile présente la forme d'un croissant de lune avec dans l'arc intérieur du croissant une fente en V. En ramenant l'élément mobile contre le boîtier de la pompe, l'arête de la tête de piston pénètre dans la fente en V qui ainsi la retient contre le poussoir. Cette solution nécessite que la tête de seringue soit déjà alignée avec la fente en V de l'élément mobile.

Dans une autre solution courante, les moyens de retenue sont constitués par deux bras. Ces bras, qui sont parallèles à la tête de piston de la seringue, sont montés sur des axes pivotants, parallèles à l'axe principal de la seringue. Lorsque la seringue est mise en place dans le pousse seringue, ces bras sont écartés en les faisant pivoter vers l'extérieur, la tête de piston est placée contre le poussoir et les bras sont relâchés pour qu'ils retournent en position fermée bloquant ainsi la tête de piston entre eux et le poussoir.

Tels quels, les bras ne peuvent bloquer efficacement que des têtes de seringue d'une épaisseur définie. Autrement dit, les pousse seringues correspondants ne peuvent être utilisés que pour un type de seringue ou des seringues d'un seul fabricant sous réserve que ces dernières aient toutes une tête de piston de même épaisseur. L'utilisation de ces bras pivotants avec des têtes de piston plus épaisses n'est pas possible, les bras ne pouvant pas se refermer, tandis que l'utilisation avec des têtes de piston plus fines est dangereuse dans la mesure où même un très faible effet de siphonage peut avoir de graves conséquences, notamment pour les solutions délivrées à très faible vitesse.

Il existe donc des bras qui non seulement pivotent pour s'ouvrir, mais en plus suivent un mouvement de translation le long de l'axe de la seringue de sorte qu'ils peuvent s'adapter à l'épaisseur de la tête de piston. En appuyant sur un levier, on provoque tout d'abord la translation des bras, ce qui tend à les éloigner du poussoir, puis un mouvement de rotation des bras, ce qui tend à les faire pivoter vers l'extérieur en les écartant l'un de l'autre. Une fois la tête de piston placée contre le poussoir, le levier est relâché, les bras se referment jusqu'à toucher la tige du piston de la seringue, puis ils reculent en direction du poussoir jusqu'à toucher la tête de piston de la seringue.

On connaît un tel dispositif de retenue par exemple du document US 6,428,509 B1. Ce dispositif comprend un premier mécanisme de pivotement qui provoque le pivotement des bras et un deuxième mécanisme qui lui provoque la translation des bras. Le mécanisme de pivotement est constitué essentiellement de trois roues dentées placées en série, la première transmettant le mouvement de rotation du levier d'actionnement à une deuxième roue dentée qui est solidaire du premier bras, la deuxième roue dentée transmettant à son tour le mouvement de rotation à une troisième roue dentée solidaire du deuxième bras. Dans le mécanisme de translation, les axes de rotation des bras sont poussés contre une plaque d'actionnement qui présente sur son autre face une saillie. Le levier d'actionnement est solidaire d'une plaque de commande présentant une rampe, la saillie de la plaque d'actionnement prenant appui sur la plaque de commande du côté de la rampe. Lorsque le levier pivote, cette plaque de commande se déplace jusqu'à ce que la saillie glisse contre la rampe provoquant le déplacement vers le bas de la plaque d'actionnement et donc la translation des bras en les éloignant du poussoir. Un crochet bloque la plaque d'actionnement lorsqu'elle a atteint une certaine distance de telle sorte qu'elle se trouve bloquée dans cette position sans pouvoir revenir en arrière. Lorsque le levier d'actionnement est ramené en arrière, les bras se referment. Ce n'est que dans les derniers degrés de pivotement du levier que le crochet retenant la plaque d'actionnement est libéré permettant à cette dernière de revenir vers le poussoir et entraînant avec elle les bras. Ce dispositif est particulièrement compliqué et nécessite de nombreuses pièces.

On connaît du document EP 1 066 846 A1 un autre dispositif de retenue avec des bras pouvant pivoter et se translater. Chaque bras est monté sur un axe portant chacun une roue dentée munie d'une gorge radiale centrale. Une roue dentée reliée indirectement à un moteur électrique est munie d'une projection radiale centrale. Cette roue dentée motrice est destinée à coopérer avec la roue dentée du premier bras, la projection radiale de la première pénétrant dans la gorge radiale centrale de la seconde. La roue dentée motrice coopère également avec une roue dentée de transmission qui transmet le mouvement de rotation de la roue motrice à la roue dentée du second bras. La roue dentée du second bras est positionnée de telle sorte que la projection radiale de la roue dentée motrice pénètre également dans sa gorge radiale centrale sans qu'elles soient pour autant en prise. Par ailleurs, la roue dentée motrice est montée sur une bague munie d'un filetage intérieur. La bague est elle-même montée sur une tige creuse filetée à l'extérieur et portant à l'une de ses extrémités une roue dentée coopérant avec le moteur. Une tige terminée par une collerette pénètre par son extrémité libre dans la tige creuse, du côté opposé à la roue dentée coopérant avec le moteur. Cette tige est libre en rotation dans la tige creuse tout en étant bloquée en translation. Cet assemblage permet un mouvement en translation de la roue dentée motrice, qui entraîne avec elle les roues dentées des bras en raison de l'engagement de la projection radiale dans les gorges radiales des roues dentées des bras. Le mouvement de translation est limité d'un côté par la roue dentée coopérant avec le moteur et de l'autre par la collerette. Un jeu de ressorts favorise la translation de la roue motrice au détriment de son pivotement qui ne se déclenche que lorsque la roue motrice ne peut plus se déplacer en translation. Ici encore, le mécanisme fait appel à de nombreuses pièces et sa conception est particulièrement compliquée.

Par ailleurs, on connaît du document US 2004/0116893 A1 un dispositif de retenue du cylindre de la seringue qui prévoit dans une de ses variantes trois bras pouvant pivoter dans un plan radial vers le centre de l'adaptateur. Ces bras ne peuvent cependant pas se déplacer en direction axiale.
Le document WO 01/08726 A1 décrit un dispositif tel que divulgué dans le préambule de la revendication 1.

L'objectif de l'invention est donc de développer un mécanisme selon le préambule qui soit d'une conception plus simple en faisant appel à un nombre de pièces moins important. Un autre objectif de l'invention est de diminuer le nombre de pièces devant subir une translation afin de limiter les frottements et le risque de coinçage de certaines pièces.

Cet objectif est atteint avec le dispositif conforme à l'invention dans lequel les moyens pour transformer et transmettre une partie du mouvement de l'organe de commande en un mouvement de translation d'au moins un pignon sont bloqués en translation. On limite ainsi le mouvement de translation aux seuls pignons solidaires des roues, ce qui diminue le risque de blocage.

Dans un mode de réalisation privilégié de l'invention, les moyens pour transformer et transmettre une partie du mouvement de l'organe de commande en un mouvement de translation d'au moins un pignon et les moyens pour transformer et transmettre une partie du mouvement de l'organe de commande en un mouvement de rotation d'au moins un pignon sont confondus. Le nombre de pièces est donc considérablement diminué.

Dans la perspective d'une diminution du nombre de pièces, il est préférable d'engrener les pignons solidaires des bras l'un dans l'autre de sorte que la rotation d'un des pignons entraîne la rotation en sens inverse de l'autre pignon. On évite ainsi de devoir interposer un pignon supplémentaire entre le deuxième pignon et les moyens pour transmettre et transformer le mouvement de l'organe de commande en un mouvement de rotation d'au moins un bras, pour inverser le mouvement de rotation par rapport au premier pignon.

Afin de solidariser en translation les pignons solidaires des bras, il est préférable de munir l'un des pignons sur ses faces latérales de flasques. Ainsi lorsque l'un des pignons sera soumis à un mouvement de translation, il entraînera l'autre aussi bien dans le sens de l'éloignement des bras que dans le sens retour.

Dans un mode de réalisation privilégié de l'invention, les moyens pour transformer et transmettre une partie du mouvement de l'organe de commande en un mouvement de translation d'au moins un pignon et les moyens pour transformer et transmettre une partie du mouvement de l'organe de commande en un mouvement de rotation d'au moins un pignon sont constitués d'une denture hélicoïdale. La forme hélicoïdale de la denture permet en la déplaçant de donner à la fois un mouvement de translation et un mouvement de rotation à une denture coopérant avec elle. Pour des raisons de simplicité et d'économie de place, il est préférable que la denture hélicoïdale soit montée sur un segment de pignon, de préférence orientée vers l'intérieur dudit segment de pignon.

Il est préférable de munir le pignon qui coopère avec les moyens de transformation et de transmission d'une denture hélicoïdale compatible avec la denture hélicoïdale des moyens de transformation et de transmission, et de faire coopérer ces deux dentures hélicoïdales entre elles. Il est préférable que ces deux dentures soient directement engrenées l'une dans l'autre afin d'éviter l'interposition de pièces supplémentaires.

Pour des raisons de simplicité, il est préférable de munir les deux pignons solidaires des bras de dentures hélicoïdales inversées. Autrement dit, la denture hélicoïdale du pignon coopérant avec la denture des moyens de transformation et de transmission sert aussi bien à la transmission du mouvement par les moyens de transformation et de transmission qu'à la transmission du mouvement de rotation au deuxième pignon solidaire du deuxième bras. Dans la pratique, un mouvement de rotation de la denture hélicoïdale du pignon hélicoïdal sous l'effet d'un mouvement de rotation de l'organe de commande entraîne un mouvement de translation et de rotation du pignon du premier bras qui coopère avec le pignon hélicoïdal. Ce pignon solidaire du premier bras transmet à son tour le mouvement de translation et de rotation au second pignon solidaire du second bras.

Pour éviter des frottements trop importants tout en garantissant un équilibre entre translation et rotation, les dentures hélicoïdales sont de préférence inclinées d'environ 45°.

Afin de garantir dans le sens de l'ouverture que la translation se fasse avant la rotation et dans le sens du retour en position de repos ce soit la rotation qui se fasse avant la translation, des moyens de poussée sont prévus pour pousser l'un au moins des pignons en position éloignée, ces moyens étant constitués de préférence d'un ressort de poussée, l'effet de ces moyens de poussée étant de préférence suffisamment puissant pour favoriser, dans le sens de l'ouverture et de l'éloignement des bras, le mouvement de translation des pignons par rapport au mouvement de rotation de ces derniers. Ainsi, lorsque le pignon hélicoïdal pivote, le pignon engrené dans celui-ci tend sous l'effet du ressort de poussée à effectuer de préférence un mouvement de translation entraînant avec lui en translation l'autre pignon et donc les bras. Lorsque ce mouvement de translation est fini, la poursuite du mouvement de rotation du pignon hélicoïdal provoque la rotation du pignon engrené et donc l'ouverture des bras. Quand l'organe de commande est relâché, le ressort de poussée empêche au début la translation du pignon engrené, le forçant donc à pivoter entraînant l'autre pignon et provoquant un mouvement de fermeture des bras. Lorsque ceux-ci butent sur la tige du piston de la seringue ou que leurs extrémités se touchent, la rotation du pignon engrené est bloquée et seul le mouvement de translation est possible. Ce mouvement de rétractation se poursuit jusqu'à ce que les bras butent contre la tête de piston de la seringue ou le poussoir du pousse seringue.

Afin de forcer le dispositif de retenue à revenir automatiquement en positon de repos ou dans la position la plus proche possible de cette position extrême, des moyens de rappel sont prévus pour rappeler le dispositif de retenue en position de repos dans laquelle les bras sont le plus refermés possible et le plus proches possible du poussoir. Afin d'assurer un bon fonctionnement, l'effet des moyens de rappel est de préférence supérieur à l'effet des moyens de poussées, de sorte que le dispositif tend à revenir en position de repos lorsque l'organe d'actionnement est sans effet.

L'organe de commande peut être solidaire d'un levier de commande extérieur et/ou d'un moyen de transmission commandé par le pousse seringue. Ainsi, il est possible d'actionner le dispositif de retenue soit manuellement soit automatiquement.

Il est préférable de prévoir des moyens pour permettre à l'organe de commande de retourner en position de repos même si le dispositif de retenue reste bloqué dans une position intermédiaire entre la position d'ouverture et la position de repos. On évite ainsi d'endommager le dispositif de retenue lorsque l'organe de commande revient en position de repos alors que le dispositif de retenue retient une tête de piston et ne peut donc pas revenir en position de repos complet.

Un exemple de réalisation de l'invention est présenté ci-dessous à l'aide des figures qui montrent:

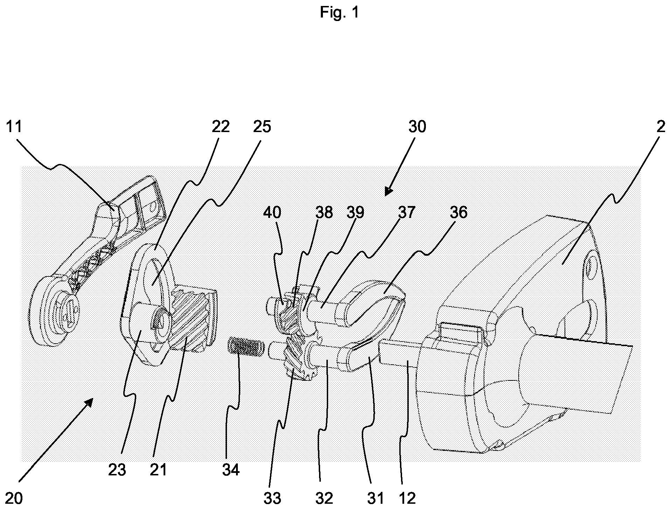

- Figure 1 :: vue éclatée du dispositif de retenue selon l'invention ;
- Figure 2 :: vue de derrière du dispositif en position fermée ;
- Figure 3 :: vue en perspective du dispositif de la figure 2 ;
- Figure 4 :: vue de derrière du dispositif en position ouverte ;
- Figure 5 :: vue en perspective du dispositif de la figure 4 ;
- Figure 6 :: vue en coupe du dispositif en position fermée selon la figure 2 ;
- Figure 7 :: vue en coupe du dispositif en position ouverte selon la figure 4.

Le dispositif de retenue (1) selon l'invention est constitué essentiellement de trois éléments principaux : un organe de commande (10), un dispositif de transformation et de transmission (20) et un mécanisme de transfert du mouvement au bras (30). L'ensemble du mécanisme, à l'exception des bras, est enfermé dans un boîtier formé d'un couvercle (2) et d'un fond (3).

Les bras (31, 36) sont placés chacun sur un axe (32, 37) parallèle à l'axe de la seringue. Chacun des bras porte un pignon à denture hélicoïdale (33, 38). La denture de ces pignons est inclinée de préférence à 45°. Les deux pignons (33, 38) sont conçus pour coopérer ensemble, l'inclinaison de leurs dentures est donc inversée. Afin de solidariser en translation les deux pignons (33, 38), l'un des deux, ici le pignon supérieur (38) est muni sur ses deux faces latérales d'un flasque (39, 40). Les faces latérales des dents du pignon inférieur (33) sont donc placées entre les deux flasques (39, 40), ce qui force le pignon supérieur (38) à se déplacer en même temps que le pignon supérieur (33).

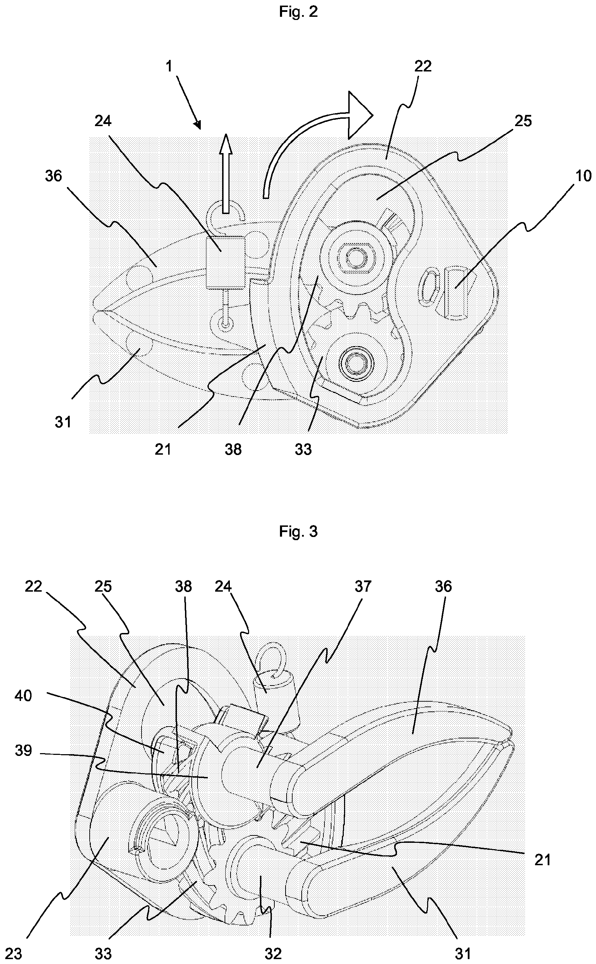

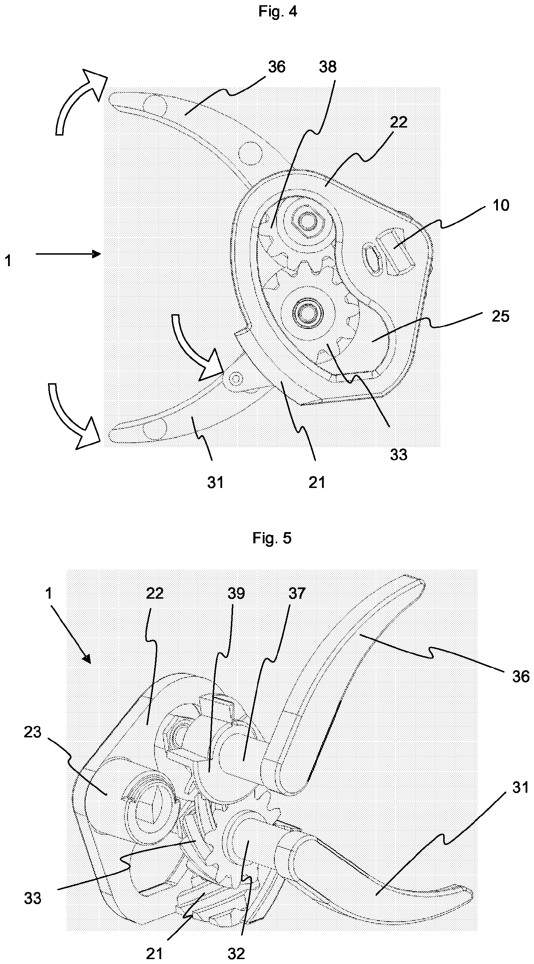

Par ailleurs, les bras (31, 36) et donc les axes de rotation (32, 37) peuvent pivoter en sens inverse (en raison la prise entre les deux pignons (33, 38)) entre une position « fermée » où les extrémités libres de bras (31, 36) se touchent (figure 3) et une position « ouverte » dans laquelle les bras (31, 36) sont écartés au maximum (figure 5). Les bras (31, 36) passent de l'une à l'autre position sous l'effet d'une rotation relative des pignons (33, 38). Autrement dit, en faisant pivoter le pignon inférieur (33), on fait pivoter le pignon supérieur (38), ce qui provoque l'ouverture ou la fermeture des bras (31, 36).

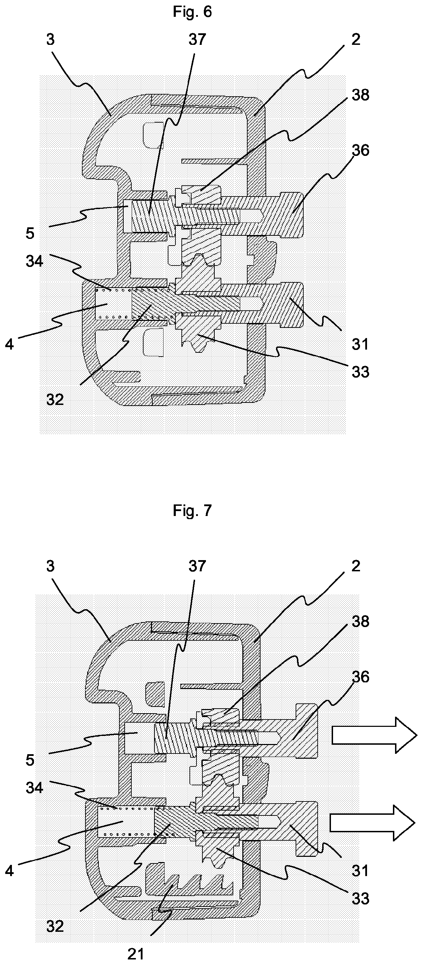

Les axes de rotation (32, 37) des bras (31, 36) sont logés dans le boîtier (2, 3) dans des cavités cylindriques (4, 5) réalisées dans le fond du boîtier (3). Les bras (31, 36) traversent des ouvertures correspondantes réalisées dans le couvercle du boîtier (2). Les cavités cylindriques (4, 5) et les ouvertures correspondantes sont disposées de telle sorte que les pignons (33, 38) soient engrenés l'un dans l'autre. Les ensembles bras/axes de rotation (31/32, 36/37) peuvent coulisser dans le boîtier entre une position « plaquée » (voir figure 6) et une position « sortie » (voir figure 7). Dans la position « plaquée », les bras (31, 36) sont plaqués ou presque contre le poussoir, c'est-à-dire contre le couvercle du boîtier (2). Dans le cas représenté, les axes de rotation sont enfoncés dans les cavités cylindriques (4, 5) du fond du boîtier (3) et les pignons (33, 38) sont en appui contre une première butée, par exemple le bord des cavités cylindriques (4, 5). En position « sortie », les bras sont dans la position la plus éloignée du poussoir, ce qui se caractérise par la position des pignons (33, 38) en appui contre une seconde butée, par exemple le couvercle (2) du boîtier. Un ressort (34) tend à repousser l'axe de rotation inférieur (32) en position « sortie ».

Le mécanisme de transformation et de transmission (20) est constitué essentiellement d'un pignon hélicoïdal à denture hélicoïdale intérieure (21). Cette denture est inclinée de préférence à 45°, comme celle des pignons (33, 38). La denture hélicoïdale (21) est destinée à coopérer avec le pignon inférieur (33) du bras inférieur (31). Elle est placée sur un support (22) ayant en son centre une fente (25) suffisamment grande pour laisser passer librement les axes de rotation (32, 37) portant les bras (31, 36). On obtient ainsi un dispositif très compact. Du côté opposé à la denture hélicoïdale (21), le support (22) est muni d'une bague (23) qui est destinée à recevoir l'organe de commande (10). Le mécanisme de transformation et de transmission pivote autour de l'axe de cette bague (23). Cette bague est logée dans le boîtier (2, 3) de sorte à pouvoir pivoter entre deux positions extrêmes tout en étant bloquée en translation. Un ressort (24) tend à maintenir ou à ramener le mécanisme de transformation et de transmission dans sa position de repos (voir figure 2). Cette position correspond à la position « plaquée » et « fermée » des bras (31, 36). Lorsque l'organe de commande (10) transmet au dispositif de transformation et de transmission un mouvement de rotation suffisamment puissant pour contrer l'effet du ressort (24), ce mouvement de rotation est transmis par la bague (23) au support (22) et enfin à la denture hélicoïdale (21). Cette dernière se déplace jusqu'à une position extrême correspondant à la position « sortie » et « ouverte » des bras (31, 36). Le ressort (24) est placé de préférence en traction entre le boîtier (2) et un point proche de la denture hélicoïdale (21).

L'organe de commande (10) peut être constitué par un levier (11) et/ou une tige de commande (12) commandée par le pousse seringue lui-même. Il est ainsi possible au choix d'actionner le dispositif soit manuellement soit de façon automatisée. Dans les deux cas, la liaison entre l'organe de commande (10) et la bague de transmission (23) est telle qu'elle permet au dispositif de retenue de ne pas revenir dans sa position de repos complet, c'est-à-dire les bras entièrement fermés et complètement plaqués contre le poussoir. Pour cela, la tige de commande (12) n'est pas entièrement solidaire de la bague (23). Elle peut pivoter dans une ouverture (25) lui laissant un certain jeu qui sera expliqué plus bas.

Le dispositif de retenue de l'invention fonctionne de la façon suivante : en position de repos, le ressort (24) tend à ramener la denture hélicoïdale (21) en position de repos. Le pignon inférieur (33) est engrené dans la denture hélicoïdale de telle sorte qu'il se trouve plaqué contre l'ouverture de la cavité cylindrique (4), tout comme le pignon supérieur (38) est plaqué contre l'ouverture de la cavité cylindrique (5). Le ressort (34) tend certes à repousser le pignon inférieur (33) en position « sortie », mais la force de ce ressort de poussée (34) est choisie de telle sorte que son effet soit plus faible que l'effet du ressort de rappel (24). Les pignons (33, 38) sont donc bloqués dans la position dans laquelle les bras (31, 36) sont plaqués contre le poussoir (2). Dans cette position, les bras (31, 36) sont en position « fermée », c'est-à-dire que leurs extrémités libres se touchent (voir figure 3).

Lorsque l'organe de commande (10) pivote, dans le sens opposé à l'effet du ressort de rappel (24), la denture hélicoïdale pivote vers le bas (considéré par exemple aux figures 2 et 4). Elle entraîne avec elle le pignon inférieur (33) qui est en prise avec elle. Le ressort de poussée (34) tend à repousser le pignon (33) axialement en position « sortie ». La forme hélicoïdale des dentures des pignons (21, 33) permet a priori soit de transformer le mouvement de rotation du pignon hélicoïdal (21) en un mouvement de translation du pignon inférieur (33) soit de transmettre le mouvement de rotation au pignon inférieur (33). En choisissant la force du ressort (34) de telle sorte que son effet surpasse les forces de frottement dues au coulissement des dents du pignon inférieur contre la denture du pignon hélicoïdal, on favorise le mouvement de translation par rapport au mouvement de rotation. Le pignon inférieur (33) va donc se déplacer en translation le long de son axe de rotation jusqu'à ce qu'il s'arrête contre une butée, par exemple le couvercle (2) du boîtier.

Arrivé dans cette position, le mouvement de rotation du pignon hélicoïdal ne peut plus être transformé en mouvement de translation : il est donc transmis au pignon inférieur (33) sous forme de rotation. Le pignon inférieur (33) exécute donc tout d'abord un mouvement de translation puis un mouvement de rotation. Le bras inférieur (31) dont il est solidaire exécute donc un mouvement qui l'éloigne du poussoir (2) jusqu'à sa position « sortie » puis qui l'écarte de sa position fermée jusqu'à sa position « ouverte ».

Le pignon supérieur (38) étant solidaire en translation du pignon inférieur (33) en raison des flasques (39, 40) qui enserrent les dents de ce dernier, il suit tout d'abord le même mouvement de translation puis le même mouvement de rotation, mais en sens opposé. Le bras supérieur (36) est donc tout d'abord éloigné du poussoir puis écarté de sa position fermée en direction opposée au mouvement du bras inférieur (31).

Lorsque l'organe de commande retourne en position de repos, le ressort (24) tend à ramener le pignon hélicoïdal (21) en position de repos en le faisant pivoter vers le haut (par rapport aux figures 2 et 4). Le ressort (34) tendant à maintenir le pignon (33) en position « sortie », le pivotement du pignon hélicoïdal (21) provoque le pivotement du pignon inférieur (33) et donc parallèlement du pignon supérieur (38). Ceci se traduit par le retour en position fermée des bras (31, 36) jusqu'à ce qu'ils butent contre la tige de piston de la seringue (si elle est suffisamment épaisse) ou jusqu'à ce que leurs extrémités libres se rencontrent en position « fermée ». Dans les deux cas, le mouvement de rotation des pignons (33, 38) est bloqué. Le mouvement de rotation du pignon hélicoïdal (21) se transforme alors en un mouvement de translation du pignon inférieur (33) par rapport au pignon hélicoïdal (21) contre l'effet du ressort de poussée (34). Sous l'effet de ce mouvement de translation, les bras rentrent dans le boîtier (2) jusqu'à ce qu'ils butent contre la tête de piston de la seringue. Dans cette position qui ne correspond pas exactement à la position de repos, la rotation du pignon hélicoïdal est bloquée. Afin d'éviter un endommagement du dispositif, il est donc préférable de prévoir un jeu dans la bague de transmission (24) qui permette à l'organe de commande (10) de retourner dans sa position de repos sans que le dispositif de retenue soit lui-même dans sa position de repos. Lorsque le dispositif de commande entamera un nouveau mouvement de rotation en vue par exemple de libérer la tête de piston de la seringue, le début de ce mouvement de rotation n'aura pas d'effet sur le pignon hélicoïdal et donc sur les bras.

Le dispositif selon l'invention ne comprend donc que très peu de pièces dont seulement un tout petit nombre est soumis à un mouvement de translation. Le dispositif de retenue de l'invention est donc beaucoup plus fiable et robuste à l'usage et son coût de fabrication peut être diminué par rapport à celui des dispositifs de l'état de la technique.

Au lieu d'un pivot hélicoïdal (21/22) il est également possible de prévoir une denture hélicoïdale plane qui soit soumise non pas à un mouvement de rotation, mais à un mouvement de translation parallèle à son plan principal. De même, il est possible de placer la denture hélicoïdale (21) non pas vers l'intérieur du pivot (22) mais vers l'extérieur en plaçant les pignons solidaires de bras à l'extérieur du pivot (22). Un tel dispositif est cependant moins compact que celui présenté précédemment.

### Liste des références :

- 1: Dispositif de retenue
- 2: Couvercle de boîtier
- 3: Fond du boîtier
- 4: Cavité cylindrique inférieure
- 5: Cavité cylindrique supérieure
- 10: Organe de commande
- 11: Levier de commande manuelle
- 12: Tige de commande automatique
- 20: Dispositif de transformation et de transmission
- 21: Denture hélicoïdale
- 22: Support
- 23: Bague
- 24: Ressort de rappel
- 25: Fente
- 30: Dispositif de transmission du mouvement
- 31: Bras inférieur
- 32: Axe de rotation inférieur
- 33: Pignon inférieur
- 34: Ressort de poussée
- 36: Bras supérieur
- 37: Axe de rotation supérieur
- 38: Pignon supérieur
- 39: Flasque
- 40: Flasque

## Revendications

1. Dispositif de retenue (1) pour bloquer la tête de piston d'une seringue sur le poussoir (2) d'un pousse seringue, le dispositif étant muni de bras (31, 36) solidaires chacun d'un pignon (33, 38) pouvant pivoter autour de son axe et pouvant se déplacer en translation en entraînant le bras correspondant dans un même mouvement entre une position de repos dans laquelle les bras sont « fermés » et « plaqués » contre le poussoir (2) et une position d'ouverture dans laquelle les bras sont « ouverts » et « éloignés » du poussoir (2), la translation étant réalisée avant la rotation lors du mouvement vers la position d'ouverture ou inversement vers la position de repos, le dispositif de retenue comprenant en outre un organe de commande (10) et des moyens (20) pour transformer une partie du mouvement de l'organe de commande (10) en le transmettant à au moins un pignon (33) sous la forme d'un mouvement de translation et des moyens (20) pour transformer une partie du mouvement de l'organe de commande (10) en le transmettant à au moins un pignon (33) sous la forme d'un mouvement de rotation, **caractérisé en ce que**
- les moyens (20) pour transformer et transmettre une partie du mouvement de l'organe de commande (10) en un mouvement de translation d'au moins un pignon (33) sont bloqués en translation, et **en ce que**
- les moyens (20) pour transformer et transmettre une partie du mouvement de l'organe de commande (10) en un mouvement de translation d'au moins un pignon (33) et les moyens (20) pour transformer et transmettre une partie du mouvement de l'organe de commande (10) en un mouvement de rotation d'au moins un pignon (33) sont confondus,
- lesdits moyens (20) étant constitués d'une denture hélicoïdale (21).

2. Dispositif selon la revendication précédente, **caractérisé en ce que** les pignons (33, 38) solidaires des bras (31, 36) sont engrenés l'un dans l'autre de sorte que la rotation d'un des pignons (33) entraîne la rotation en sens inverse de l'autre pignon (38).

3. Dispositif selon la revendication précédente, **caractérisé en ce que** l'un des pignons (38) est muni sur ses faces latérales de flasques (39, 40) pour solidariser en translation les deux pignons (33, 38).

4. Dispositif de retenue selon l'une des revendications précédentes, **caractérisé en ce que** la denture hélicoïdale (21) est montée sur un pignon (22), en étant placée de préférence vers l'intérieur du pignon (22).

5. Dispositif selon la revendication précédente, **caractérisé en ce que** le pignon (33) qui coopère avec les moyens de transformation et de transmission (20) est muni d'une denture hélicoïdale compatible avec la denture hélicoïdale (21) des moyens de transformation et de transmission (20), et **en ce que** les deux dentures hélicoïdales (21, 33) coopèrent entre elles, lesdites dentures (21, 33) étant de préférence engrenées l'une dans l'autre.

6. Dispositif selon la revendication précédente, **caractérisé en ce que** les deux pignons (33, 38) solidaires des bras (31, 36) sont munis de dentures hélicoïdales inversées.

7. Dispositif selon l'une des revendications 4 à 6, **caractérisé en ce que** les dentures hélicoïdales sont inclinées d'environ 45°.

8. Dispositif selon l'une des revendications précédentes, **caractérisé en ce que** des moyens de poussée sont prévus pour pousser l'un au moins des pignon (33) en position éloignée, ces moyens étant constitués de préférence d'un ressort de poussée (34), l'effet de ces moyens de poussée étant de préférence suffisamment puissant pour favoriser, dans le sens de l'ouverture et de l'éloignement des bras (31, 36), le mouvement de translation des pignons (33, 38) par rapport au mouvement de rotation de ces derniers.

9. Dispositif selon l'une des revendications précédentes, **caractérisé en ce que** des moyens de rappel sont prévus pour rappeler le dispositif de retenue en position de repos ou dans une position la plus proche possible de cette position extrême, dans laquelle les bras (31, 36) sont le plus refermés possible et le plus proches possible du poussoir (2).

10. Dispositif de retenue selon les revendications 8 et 9, **caractérisé en ce que** l'effet des moyens de rappel (24) est supérieur à l'effet des moyens de poussée (34) de sorte que le dispositif tend à revenir en position de repos lorsque l'organe d'actionnement est sans effet.

11. Dispositif de retenue selon l'une des revendications précédentes, **caractérisé en ce que** l'organe de commande (10) est solidaire d'un levier de commande extérieur et/ou d'un moyen de transmission commandé par le pousse seringue.

12. Dispositif de retenue selon l'une des revendications précédentes, **caractérisé en ce que** des moyens sont prévus pour permettre à l'organe de commande de retourner en position de repos même si le dispositif de retenue reste bloqué dans une position intermédiaire entre la position d'ouverture et la position de repos.

## Patentansprüche

1. Haltevorrichtung (1) zum Verriegeln des Kolbenkopfes einer Spritze am Spritzenschieber (2) einer Spritzenpumpe, wobei die Vorrichtung mit Armen (31, 36) versehen ist, die jeweils mit einem Ritzel (33, 38) gekoppelt sind, das um seine Achse drehbar und translatorisch bewegbar ist und dabei den entsprechenden Arm in der gleichen Bewegung zwischen einer Ruheposition, in der die Arme "geschlossen" und "gedrückt" gegen den Spritzenschieber (2) sind, und einer Öffnungsposition, in der die Arme "offen" und "entfernt" vom Spritzenschieber (2) sind, antreiben, wobei die Translation vor der Drehung während der Bewegung in Richtung der Öffnungsposition oder umgekehrt in Richtung der Ruheposition durchgeführt wird, wobei die Haltevorrichtung ferner ein Steuerelement (10) und Mittel (20) zum Umwandeln eines Teils der Bewegung des Steuerelements (10) durch Übertragen auf mindestens ein Ritzel (33) in Form einer Translationsbewegung und Mittel (20) zum Umwandeln eines Teils der Bewegung des Steuerelements (10) durch Übertragen auf mindestens ein Ritzel (33) in Form einer Drehungsbewegung umfasst, **dadurch gekennzeichnet, dass**
- die Mittel (20) zum Umwandeln und Übertragen eines Teils der Bewegung des Steuerelements (10) in eine Translationsbewegung mindestens eines Ritzels (33) gegen Translation gesichert sind, und dass
- die Mittel (20) zum Umwandeln und Übertragen eines Teils der Bewegung des Steuerelements (10) in eine Translationsbewegung mindestens eines Ritzels (33) und die Mittel (20) zum Umwandeln und Übertragen eines Teils der Bewegung des Steuerelements (10) in eine Rotationsbewegung mindestens eines Ritzels (33) ineinander vereint sind,
- wobei die Mittel (20) aus einer Helikoidalverzahnung (21) bestehen.

2. Vorrichtung nach dem vorhergehenden Anspruch, **dadurch gekennzeichnet, dass** die mit den Armen (31, 36) gekoppelten Ritzel (33, 38) so ineinandergreifen, dass die Drehung eines der Ritzel (33) die entgegengesetzte Drehung des anderen Ritzels (38) bewirkt.

3. Vorrichtung nach dem vorhergehenden Anspruch, **dadurch gekennzeichnet, dass** eines der Ritzel (38) auf seinen Seitenflächen mit Flanschen (39, 40) zum translatorischen Koppeln der beiden Ritzel (33, 38) versehen ist.

4. Haltevorrichtung nach einem der vorstehenden Ansprüche, **dadurch gekennzeichnet, dass** die Helikoidalverzahnung (21) auf einem Ritzel (22) gelagert ist, wobei sie vorzugsweise zur Innenseite des Ritzels (22) hin angeordnet ist.

5. Vorrichtung nach dem vorhergehenden Anspruch, **dadurch gekennzeichnet, dass** das Ritzel (33), das mit den Mitteln zum Umwandeln und Übertragen (20) zusammenwirkt, mit einer Helikoidalverzahnung versehen ist, die mit der Helikoidalverzahnung (21) der Mittel zum Umwandeln und Übertragen (20) kompatibel ist, und dass die beiden Helikoidalverzahnungen (21, 33) miteinander zusammenwirken, wobei die Verzahnungen (21, 33) vorzugsweise ineinander greifen.

6. Vorrichtung nach dem vorhergehenden Anspruch, **dadurch gekennzeichnet, dass** die beiden Ritzel (33, 38), die mit den Armen (31, 36) gekoppelt sind, mit umgekehrten Helikoidalverzahnungen versehen sind.

7. Vorrichtung nach einem der Ansprüche 4 bis 6, **dadurch gekennzeichnet, dass** die Helikoidalverzahnungen um etwa 45° geneigt sind.

8. Vorrichtung nach einem der vorstehenden Ansprüche, **dadurch gekennzeichnet, dass** Schubmittel vorgesehen sind, um mindestens eines der Ritzel (33) in die entfernte Position zu drücken, wobei diese Mittel vorzugsweise aus einer Druckfeder (34) bestehen, wobei die Wirkung dieser Druckmittel vorzugsweise ausreichend stark ist, um in Richtung des Öffnens und Entfernens der Arme (31, 36) die Translationsbewegung der Ritzel (33, 38) in Bezug auf die Drehbewegung dieser zu begünstigen.

9. Vorrichtung nach einem der vorstehenden Ansprüche, **dadurch gekennzeichnet, dass** Rückstellmittel vorgesehen sind, um die Haltevorrichtung in die Ruheposition oder in eine Position, die dieser extremen Position so nahe wie möglich kommt, in der die Arme (31, 36) so geschlossen wie möglich und so nah wie möglich am Schieber (2) sind, zurück zu bringen.

10. Haltevorrichtung nach den Ansprüchen 8 und 9, **dadurch gekennzeichnet, dass** die Wirkung der Rückstellmittel (24) größer ist als die Wirkung der Schubmittel (34), so dass die Vorrichtung dazu neigt, in die Ruheposition zurückzukehren, wenn das Betätigungsorgan unwirksam ist.

11. Haltevorrichtung nach einem der vorstehenden Ansprüche, **dadurch gekennzeichnet, dass** das Steuerelement (10) mit einem externen Steuerhebel und/oder einem von der Spritzenpumpe gesteuerten Übertragungsmittel gekoppelt ist.

12. Haltevorrichtung nach einem der vorstehenden Ansprüche, **dadurch gekennzeichnet, dass** Mittel vorgesehen sind, die es dem Steuerelement ermöglichen, in die Ruheposition zurückzukehren, auch wenn die Haltevorrichtung in einer Zwischenposition zwischen der Öffnungs- und der Ruheposition verriegelt bleibt.

## Claims

1. Holding device (1) for locking the piston head of a syringe on the pusher (2) of a syringe pump, the device being equipped with arms (31, 36) coupled each with a pinion (33, 38) pivotable about its axis and movable in translation so as to drive the corresponding arm in a same movement between a rest position in which the arms are "closed" and "pressed" against the pusher (2) and an open position in which the arms are "opened" and "removed" from the pusher (2), the translation being performed before the rotation during the movement toward the open position or in reverse order toward the rest position, the holding device further comprising a control member (10) and means (20) for converting a portion of the movement of the control member (10) by transmitting it to at least one pinion (33) in the form of a translation movement and means (20) for converting a portion of the movement of the control member (10) by transmitting it to at least one pinion (33) in the form of a rotation movement, **characterised in that**
- the means (20) for converting and transmitting a portion of the movement of the control member (10) into a translation movement of at least one pinion (33) are locked in translation, and **in that**
- the means (20) for converting and transmitting a portion of the movement of the control member (10) into a translation movement of at least one pinion (33) and the means (20) for converting and transmitting a portion of the movement of the control member (10) into a rotation movement of at least one pinion (33) are merged,
- wherein said means (20) are constituted by a helical set of teeth (21).

2. Device according to the preceding claim, **characterised in that** the pinions (33, 38) coupled with the arms (31, 36) mesh with each other so that the rotation of one of the pinions (33) causes the rotation of the other pinion (38) in the opposed direction.

3. Device according to the preceding claim, **characterised in that** one of the pinions (38) is equipped on its lateral faces with flanges (39, 40) to couple the two pinions with each other in translation.

4. Holding device according to one of the preceding claims, **characterised in that** the helical set of teeth (21) is mounted on a pinion (22), the set of teeth being then preferably located toward the inside of the pinion (22).

5. Device according to the preceding claim, **characterised in that** the pinion (33) that cooperates with the converting and transmitting means (20) is equipped with a helical set of teeth compatible with the helical set of teeth (21) of the converting and transmitting means (20), and **in that** the two helical sets of teeth (21, 33) cooperate with each other, said sets of teeth (21, 33) preferably meshing with each other.

6. Device according to the preceding claim, **characterised in that** the two pinions (33, 38) coupled with the arms (31, 36) are equipped with reversed helical sets of teeth.

7. Device according to one of claims 4 to 6, **characterised in that** the helical sets of teeth are slanted at an angle of about 45°.

8. Device according to one of the preceding claims, **characterised in that** pushing means are provided to push at least one of the pinions (33) toward the removed position, these means being constituted preferably by a push spring (34), the action of these pushing means being preferably sufficiently strong to promote, in the direction of opening and moving the arms (31, 36) away, the translation movement of the pinion (33, 38), as compared to their rotation movement.

9. Device according to one of the preceding claims, **characterised in that** return means are provided to return the holding device to the rest position or to a position as close as possible to this extreme position, in which the arms (31, 36) are as closed as possible and as close as possible to the pusher (2).

10. Holding device according to one of claims 8 and 9, **characterised in that** the action of the return means (24) is stronger than the action of the pushing means (34) so that the device tends to return to the rest position when the actuating member is not in action.

11. Holding device according to one of the preceding claims, **characterised in that** the control member (10) is coupled with an external control lever and/or transmitting means controlled by the syringe pump.

12. Holding device according to one of the preceding claims, **characterised in that** means are provided to enable the control member to return to the rest position even if the holding device remains locked in an intermediary position between the open position and the rest position.
